# EUROPEAN PATENT SPECIFICATION

(11) **EP 1 176 961 B1**
(45) Date of publication and mention of the grant of the patent: **05.11.2003**
(21) Application number: 00929696.3
(22) Date of filing: 11.05.2000
(51) Int. Cl.: A61K 31/465, A61K 9/50, A61K 9/00

(54) **NICOTINE DELIVERY SYSTEMS**
NIKOTINHALTIGE VERABREICHUNGSSYSTEME
PROCEDES D'APPORT DE NICOTINE

(30) Priority: 13.05.1999 GB 9911037
(43) Date of publication of application: 06.02.2002
(73) Proprietor: Fluid Technologies Plc, Haydock, Lancashire WA12 0JQ (GB)
(72) Inventor: MCNEIGHT, David Leslie, Wilmslow, Cheshire SK9 2LR (GB)
(74) Representative: Brand, Thomas Louis
(86) International application number: GB0001807
(87) International publication number: WO00069440

(56) References cited:
- WO-A-97/38662
- DE-A- 19 856 432
- GB-A- 2 171 906
- GB-A- 2 299 756
- K.H. BAUER, K.-H. FRöMMING, C. FüHRER: "'Pharmazeutische Technologie', p. 341 f.", 1991, GEORG THIEME VERLAG, STUTTGARD, NEW YORK

## Description

This invention relates to delivery systems for nicotine.

Nicotine is commonly taken in the form of smoking tobacco, in cigarettes, principally, cigars and pipe tobacco. To a lesser extent, tobacco, or a preparation from it, is chewed. More rarely, nowadays, is snuff taken. Smoking is declared to be injurious to health, though nicotine itself, in appropriate quantity, is not harmful in the way smoking is, which is due to components other than the nicotine in cigarette smoke and may even be beneficial - it is reported on numerous occasions as aiding concentration.

Though some question it, nicotine is generally regarded as addictive - certainly, increasing taxes on tobacco, Government health warnings and high profile lawsuits brought against tobacco companies by those made terminally ill, or their bereaved, seem to do little to reduce consumption.

There are several products commercially available to help those wishing to quit smoking. These take the form of tablets, chewing gum and patches, all of which are intended to deliver nicotine without the generation of smoke and its associated carcinogenic or otherwise harmful components.

A problem with formulating such products is that nicotine itself is a quite volatile liquid with a boiling point as low as 123 ° - 125 °C at atmospheric pressure, and this makes it difficult to incorporate in products on account of evaporation losses during formulation and the need to seal the products against evaporation of the nicotine for a reasonable shelf life. At the same time, the nicotine must be readily released on use - in the mouth, in the case of gum or lozenge, or through the skin in the case of a patch.

The manner of injection of nicotine is by dissolving in fatty tissue. Nicotine is not readily absorbed in the gut, and no product is intended to be swallowed.

Patches are, of course, somewhat clinical, and while no doubt quite effective, not aesthetically pleasing. Gum is widely regarded as anti-social, often as much so as smoking - there is a disposal problem involved with gum which by and large its users ignore, which has led to its being outlawed in Singapore, a measure which other countries may well follow. Of all the approaches, the most aesthetically acceptable - lozenges, which leave nothing to dispose of and which can be sucked without the sometimes highly objectionable masticating movements - are perhaps the most difficult to formulate, requiring usually elevated temperature processing, leading to nicotine loss through evaporation and an uncertain final dose in the lozenge, and special protection against evaporation from the finished product, if a reasonable shelf life is to be had.

Prior art includes the likes of GB 2999756 which discloses gelatinous pastilles containing nicotine. GB 2171906 discloses controlled release formulations for treating memory impairment, including formulations of nicotine. DE 19856432 discloses nanoparticles containing active agents such as nicotine for oral or transdermal application. K.H. Bauer *et al*. in "Pharmazeutische Technologie" 1991, Georg Thieme Verlag, Stuttgart, New York at page 341 discusses various microcapsules, nanoparticles and liposomes. None of these publications either suggests or discloses the use of yeast cells as reservoirs for (i.e. microcapsules containing) nicotine.

The present invention provides a nicotine delivery system that avoids problems of the prior art and which can give rise to improved products across the available range, but particularly in regard to the lozenge.

The invention comprises a delivery system for nicotine comprising nicotine encapsulated in a microcapsule system comprising yeast cells which releases the encapsulated nicotine on contact of the microcapsules with fatty tissue of the buccal cavity.

The system may comprise a mixture of cells charged with nicotine and diluent, empty cells.

The system may be presented in a solid carrier from the surface of which microcapsules are gradually released for controlled delivery.

The solid carrier may comprise a saliva-soluble or dispersible substance, and may comprise a lozenge, which may be sugar-based. The lozenge may have such a size, solubility and charge of nicotine that it delivers a dosage of nicotine, in use over a time period between 4 and 20 minutes, equivalent to that delivered by a cigarette. The lozenge may be elongate, between 5 and 20 cm in length and snappable as by having preferential snapping positions into a number of portions each capable of comfortable accommodation in the mouth.

The solid carrier may, however, comprise a chewing gum.

The system may comprise a flavouring substance, which may also be encapsulated in a microcapsule system, and may also comprise a vitamin supplement, which also may be encapsulated in a microcapsule system.

The system may be comprised in a patch.

Nicotine delivery systems according to the invention and embodiments of products including the same will now be described with reference to the accompanying drawings, in which:-
- Figure 1: is a diagrammatic illustration of a method of preparing microencapsulated nicotine;
- Figure 2: is a view of one embodiment of a lozenge product; and
- Figure 3: is a view of a second embodiment of a lozenge product.

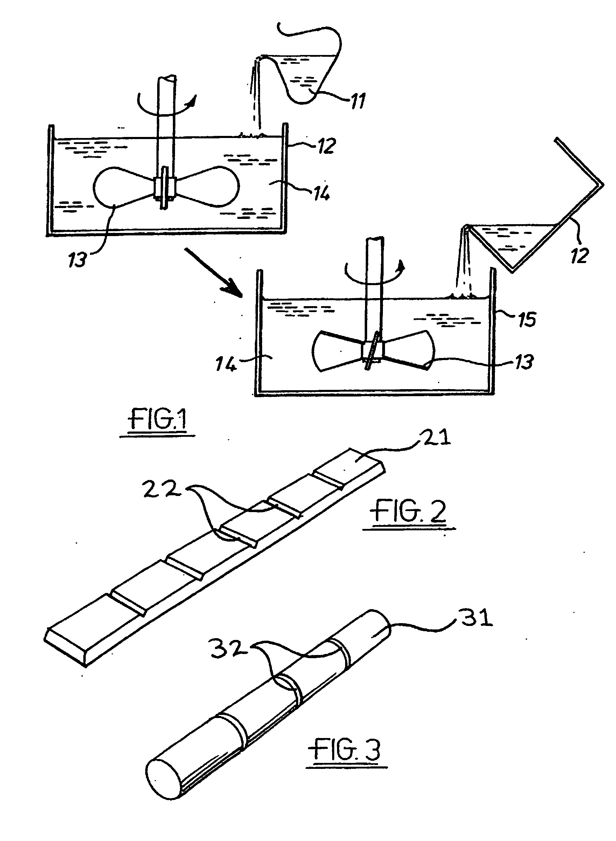

Figure 1 of the drawings illustrate a method for preparing a delivery system for nicotine comprising nicotine encapsulated in a microcapsule system which releases the encapsulated nicotine on contact of the microcapsules with a nicotine solvent.

Nicotine, in the form of liquid nicotine acid 11, is poured into a mixing vessel 12 with a paddle 13. A measured amount of nicotine is mixed into a given volume of yeast cells 14 in order to give a reasonably concentrated absorption of nicotine into each yeast cell. A suitable mix is 25 g nicotine, 50 g of yeast cells, and 100 g of water. This is stirred for 1-24 hours at about 40°C. Cells are removed by centrifugation and dried. An expected loading is between 25 and 60% by weight of nicotine into the cells, depending on the mix used.

The thus nicotine loaded yeast cells 14 are then poured from the vessel 12, in a second stage of the process, into a larger volume of yeast cells 14 in a second mixing vessel 15, also with a paddle 13, and the mixture stirred.

Thus will a desired concentration of nicotine encapsulated in yeast cells be obtained.

The mixed loaded and diluent yeast cells 14 are then incorporated into products with appropriate quantities of the yeast to give the desired nicotine dose in the product.

Two such products are illustrated in Figures 2 and 3.

Figure 2 illustrates an ingot-shaped candy bar 21 which might be some 9 or 10 cm long so as to fit into a packet such as cigarettes are sold in. The bar 21 has transverse grooves 22 enabling it to be snapped into bite-size pieces.

Figure 3 illustrates a similar product 31, this time shaped more like a cigarette, again with grooves 32 for snapping. The presentations of Figures 2 and 3 were first suggested in GB 2 299 756 A.

These products. which are quite similar to cigarettes and which may be used either as aids to quitting smoking or as cigarette substitutes where smoking is not permitted, will, by virtue of their loaded yeast content, contain an equivalent nicotine does to that delivered by smoking a cigarette.

Flavourings such for example as mint, Scotch whisky, Cognac or menthol can also be added, again encapsulated in similar fashion to the yeast, as can other beneficial agents such as vitamin supplements.

## Claims

1. A delivery system for nicotine comprising nicotine encapsulated in a microcapsule system comprising yeast cells which releases the encapsulated nicotine on contact of the microcapsules with fatty tissue of the buccal cavity.

2. A nicotine delivery system according to claim 1, comprising a mixture of cells charged with nicotine and empty diluent cells.

3. A nicotine delivery system according to either one of the preceding claims, presented in a solid carrier from the surface of which microcapsules are gradually released for controlled delivery.

4. A system according to claim 3, in which the solid carrier comprises a saliva-soluble or dispersible substance.

5. A system according to claim 4, in which the solid carrier comprises a lozenge.

6. A system according to claim 5, in which the lozenge is sugar-based.

7. A system according to either one of claims 5 or 6, having such a size, solubility and charge of nicotine that it delivers, in use over a time period between 4 and 20 minutes, an amount of nicotine equivalent to that delivered by a cigarette.

8. A system according to claim 7, in which the lozenge is elongate, between 5 and 20 cm in length and snappable as by having preferential snapping positions into a number of portions each capable of comfortable accommodation in the mouth.

9. A system according to claim 3, in which the solid carrier comprises a chewing gum.

10. A system according to any one of the preceding claims, comprising a flavouring substance.

11. A system according to claim 10, in which the flavouring substance is also encapsulated in a microcapsule system.

12. A system according to any one of the preceding claims, comprising a vitamin supplement.

13. A system according to claim 12, in which the vitamin is also encapsulated in a microcapsule system.

## Patentansprüche

1. Abgabesystem für Nicotin, umfassend Nicotin, das in ein Mikrokapselsystem eingekapselt ist, umfassend Hefezellen, die das eingekapselte Nicotin bei Kontakt der Mikrokapseln mit Fettgewebe der Mundhöhle freisetzen.

2. Nicotinabgabesystem nach Anspruch 1, umfassend ein Gemisch von Zellen, beladen mit Nicotin und leeren Diluens-Zellen.

3. Nicotinabgabesystem nach einem der vorangehenden Ansprüche, dargereicht in einem festen Träger, aus dessen Oberfläche Mikrokapseln zur kontrollierten Abgabe gestaffelt freigesetzt werden.

4. System nach Anspruch 3, worin der feste Träger eine in Speichel lösliche oder dispergierbare Substanz umfasst.

5. System nach Anspruch 4, worin der feste Träger eine Lutschtablette umfasst.

6. System nach Anspruch 5, worin die Lutschtablette auf Zucker basiert.

7. System nach einem der Ansprüche 5 oder 6, das eine solche Größe, Löslichkeit und Nicotinbeladung aufweist, dass es, bei Anwendung über eine Zeitspanne zwischen 4 und 20 Minuten, eine Nicotinmenge abgibt, die der von einer Zigarette abgegebenen entspricht.

8. System nach Anspruch 7, worin die Lutschtablette länglich, zwischen 5 und 20 cm lang und da sie bevorzugte Abbrechpositionen aufweist, in eine Anzahl von Portionen, die jeweils bequem in den Mund aufgenommen werden können, abbrechbar ist.

9. System nach Anspruch 3, worin der feste Träger einen Kaugummi umfasst.

10. System nach einem der vorangehenden Ansprüche, umfassend einen Geschmacksstoff.

11. System nach Anspruch 10, worin der Geschmacksstoff auch in ein Mikrokapselsystem eingekapselt ist.

12. System nach einem der vorangehenden Ansprüche, umfassend einen Vitaminzusatz.

13. System nach Anspruch 12, worin das Vitamin auch in einem Mikrokapselsystem eingekapselt ist.

## Revendications

1. Système d'administration de nicotine, comprenant de la nicotine encapsulée dans un système de microcapsule comprenant des cellules de levure, qui libère la nicotine encapsulée lors du contact des microcapsules avec du tissu gras de la cavité buccale.

2. Système d'administration de nicotine selon la revendication 1, comprenant un mélange de cellules chargées de nicotine et de cellules diluantes vides.

3. Système d'administration de nicotine selon l'une quelconque des revendications précédentes, présenté dans un véhicule solide à partir de la surface duquel les microcapsules sont libérées progressivement pour une administration contrôlée.

4. Système selon la revendication 3, dans lequel le véhicule solide comprend une substance soluble ou dispersable dans la salive.

5. Système selon la revendication 4, dans lequel le véhicule solide comprend une tablette.

6. Système selon la revendication 5, dans lequel la tablette est à base de sucre.

7. Système selon l'une quelconque des revendications 5 ou 6, ayant une taille, une solubilité et une charge en nicotine telles qu'il administre, lors d'une utilisation au cours d'une période comprise entre 4 et 20 minutes, une quantité de nicotine équivalente à celle administrée par une cigarette.

8. Système selon la revendication 7, dans lequel la tablette est allongée, d'une longueur comprise entre 5 et 20 cm, et sécable du fait de positions sécables préférentielles en un nombre de parties capables chacune d'une adaptation confortable dans la bouche.

9. Système selon la revendication 3, dans lequel le véhicule solide comprend un chewing-gum.

10. Système selon l'une quelconque des revendications précédentes, comprenant une substance aromatisante.

11. Système selon la revendication 10, dans lequel la substance aromatisante est également encapsulée dans un système de microcapsule.

12. Système selon l'une quelconque des revendications précédentes, comprenant un complément vitaminé.

13. Système selon la revendication 12, dans lequel la vitamine est également encapsulée dans un système de microcapsule.
